# EUROPÄISCHE PATENTANMELDUNG

(11) **EP 1 927 378 A1**
(43) Veröffentlichungstag der Anmeldung: **04.06.2008**
(21) Anmeldenummer: 07022444.9
(22) Anmeldetag: 20.11.2007
(51) Int. Cl.: A61Q 7/00, A61K 8/49, A61P 17/14, A61K 31/352

(54) **Quercetin enthaltendes Haarbehandlungsmittel und dessen Verwendung zur Vitalizierung von Haaren**

(30) Priorität: 29.11.2006 DE 102006056664
(71) Anmelder: Henkel Kommanditgesellschaft auf Aktien, 40589 Düsseldorf (DE)
(72) Erfinder: Welss, Thomas, 40591 Düsseldorf (DE); Giesen, Melanie, 47608 Geldern (DE); Schulze zur Wiesche, Erik, 20251 Hamburg (DE); Scheunemann, Volker, 21339 Lüneburg (DE)

(57) **Zusammenfassung**

Die vorliegende Erfindung betrifft Haarbehandlungsmittel, enthaltend mindestens eine Verbindung ausgewählt aus der Gruppe der Quercetine, bevorzugt Dihydroquercetin oder Dihydroquercetinhaltige Extrakte und die Verwendung dieser Mittel zur Vitalisierung von Haaren, Reaktivierung der Haarwurzel, Aktivierung von Haarfollikeln, Haarverdickung, Förderung oder Verstärkung des Haarwachstums, zur Haarkonditionierung, zur Verminderung von Haarausfall und/oder zur Behandlung von Haarausfall.

## Beschreibung

Die vorliegende Erfindung betrifft Haarbehandlungsmittel, enthaltend mindestens eine Verbindung ausgewählt aus der Gruppe der Quercetine, bevorzugt Dihydroquercetin oder Dihydroquercetin-haltige Extrakte und die Verwendung dieser Mittel.

Haarwachstum ist kein kontinuierlicher Vorgang, sondern verläuft in Wachstums- und Haarverlustschüben, die sich cyclisch wiederholen. Dieser Vorgang ist im Wesentlichen darauf zurückzuführen, dass Haarfollikelzellen einem genetisch festgelegten Zyklus von Wachstum, Regression und Ruhephase unterliegen. Der Haarfollikel ist damit das einzige Organ, das sich ständig selbst erneuert und somit einen, in Abhängigkeit von der jeweiligen Wachstumsphase, einzigartigen Metabolismus aufweist. So kommt der Metabolismus des Haarfollikels in der Ruhephase fast völlig zum Erliegen und wird mit jedem neuen Beginn eines weiteren Zyklus ebenfalls neu initiiert. Gesteuert wird dieser Zyklus von einer kleinen, hochspezialisierten Zellpopulation im Haarbulbus, den dermalen Papillenzellen, die durch ein einzigartiges komplexes System molekularer Signale, die spezifisch für jede Phase des Haarzyklus sind, das Haarwachstum kontrolliert. Im Laufe des cyclischen Vorgangs kommt es kontinuierlich zum Ausfall einzelner Haare, wobei ein Haarausfall von bis zu 100 Haaren pro Tag als physiologisch normal bezeichnet wird. Ist der komplexe Metabolismus des Haarfollikels gestört, kann es zum vorzeitigen oder übermäßigen Ausfall der Haare und schließlich zu Alopezie bis hin zu vollständiger Glatzenbildung kommen. Die Ursachen von vorzeitigem oder übermäßigem Haarausfall sind vielfältig und reichen von genetischer Veranlagung, Mängelzuständen, Vergiftungen bis hin zu Infektionen und Haarausfall im Zuge einer Chemotherapie.

ATP (Adenosintriphosphat) ist die universelle Speicherform für chemische Energie in Zellen. Bei der Abspaltung der distalen Phosphatgruppe entsteht ADP und Pi (anorganisches Phosphat). Diese Reaktion ist stark exergon, d.h. es wird Energie frei. Produziert wird ATP beim zellulären, oxidativen Abbau von Fetten, Kohlehydraten und Proteinen. ATP dient der Zelle, auch den biologisch aktiven Zellen des Haarfollikels, als Energielieferant für biochemische Synthesen und Transportvorgänge. Diese Vorgänge sind endergon, d.h. sie laufen nur unter Energiezufuhr ab. Um ihren Stoffwechsel und zelluläre Strukturen optimal aufrecht zu erhalten und zu erneuern, sind Zellen also auf eine ausreichende Versorgung mit ATP angewiesen. So ist beispielsweise die Proliferation und Differenzierung von ORS-Keratinozyten (Keratinocyten der äußeren Wurzelscheide, "outer root sheath") an die ATP-Synthese gekoppelt, da für beide Vorgänge die Biosynthese spezifischer Proteine essentielle Voraussetzung ist. Auch dermale Papillenzellen benötigen beispielsweise zur Produktion von Wachstumsfaktoren und damit zur Steuerung des Haarzyklus ATP. Daher ist eine ausreichende Versorgung des Haarfollikels mit ATP essentielle Voraussetzung für kräftiges, vitales und gesundes Haar.

Es wurde bereits eine Vielzahl von Haarbehandlungsmitteln zur Kräftigung der Haare sowie zur Verminderung von vorzeitigem Haarausfall und/oder zur Förderung des Haarwachstums entwickelt, die unterschiedlichste Wirkstoffe enthalten.

Da es zur Kräftigung der Haare sowie Verminderung von vorzeitigem Haarausfall und/oder zur Förderung des Haarwachstums wesentlich darauf ankommt, den Metabolismus des Haarfollikels positiv zu beeinflussen, muss gewährleistet werden, dass die Wirkstoffe mit den Haarfollikeln wechselwirken können. Dabei hat es sich als zweckmäßig erwiesen, entsprechende kosmetische Mittel in einer Form zu konfektionieren, die bei bestimmungsgemäßer Anwendung mit der Kopfhaut in Kontakt kommt. Typischerweise handelt es sich um Haarwässer, Shampoos oder Haarspülungen und -kuren.
Quercetin (3,3',4', 5, 7-Pentahydroxyflavon, 2-(3,4-Dihydroxyphenyl)-3, 5, 7-trihydroxy-4H-1-benzopyran-4-on)) gehört zu der Gruppe der Flavone.
Quercetin ist ein gelber Naturfarbstoff, der in vielen Pflanzen vorkommt. Beispielsweise ist Quercetin als Glycosid in Baumrinden sehr weit verbreitet. Weiterhin kommt Quercetin als Pflanzeninhaltsstoff in zahlreichen Gemüse- und Obstsorten vor (beispielsweise Salat, Tomaten, Brokkoli, Äpfel, Birnen).
Den Quercetinderivaten werden vielfältige physiologische positive Wirkungen, wie antioxidative und antikarzinogene Wirkungen, zugesprochen. Quercetine sollen beispielsweise wie Vitamin A, C und E als Radikalfänger wirken.

EP0774249 A2 beschreibt eine topische Zusammensetzung, umfassend ein erstes Flavonderivat, ausgewählt aus Eriodictyl und / oder Taxifolin und ein zweites Flavonderivat, ausgewählt aus Hesperetin und / oder Naringenin. Vorzugsweise wird die Zusammensetzung zur topischen Anwendung auf der Haut eingesetzt.

Ebenso offenbart die DE10111045 A1 eine Wirkstoffkombination für die Haut. Die Wirstoffe sind dabei eine Kombination aus Liponsäure und einem oder mehrere Wirkstoffe, ausgewählt aus der Gruppe der Flavone, Flavanone, Isoflavone bzw. Flavonoide. Vorzugsweise wird die Wirkstoffkombination zur Prophylaxe und / oder Behandlung von entzündlicher Hautzustände und / oder zum Hautschutz bei empfindlich determinierter trockener Haut, sowie bei Pigmentstörrungen eingesetzt.

Es besteht nach wie vor Bedarf an Mittel, die bei der Anwendung im Haar eine positive Wirksamkeit hinsichtlich der Haarkräftigung, des Haarausfalles bzw. Haarwachstums aufweisen.

Aufgabe der vorliegenden Erfindung war es daher ein Mittel zur Verfügung zu stellen, welches besonders wirksam Haare kräftigt sowie vorzeitigen Haarausfall vermindert und/oder den Haarwachstum fördert.

Es wurde überraschenderweise gefunden, dass dies durch ein Mittel, enthaltend mindestens eine Verbindung ausgewählt aus der Gruppe der Quercetine erreicht werden kann.

Ein erster Gegenstand der vorliegenden Erfindung ist daher ein Haarbehandlungsmittel, enthaltend mindestens eine Verbindung ausgewählt aus der Gruppe der Quercetine.
Bevorzugte Quercetine sind vorzugsweise ausgewählt aus der Gruppe, umfassend 3,3',4',5,7-Pentahydroxyflavanone, 3,3',4',5,7-Pentahydroxyflavon 3-β-glucoside (Isoquercitrin), Quercetin-7-methylether, Quercetin-3',7-dimethylether, Quercetin-3-*O*-β-D-glucofuranoside, Quercetin-7-*O-*glucoside, Quercetin-4'-*O*-glucoside, Quercetin-3-*O*-galactoside, Quercetin-3-D-xyloside, Quercetin- 3-rhamnoside (Quercitrine), Quercetin-3-rutinoside, Dihydroquercetine und Mischungen daraus.
Vorzugsweise werden die Quercetinderivate als Extrakte gewonnen. Beispielsweise kann Quercetin-Glycoside aus Rinden und Schale vieler Früchte und Gemüsearten gewonnen werden. Quercetin-7-methylether, Quercetin-3',7-dimethylether kommen beispielsweise in Rosskastanien, Zwiebel, Kamille Stiefmütterchen, Eichen und Rosen vor.
Daher ist der Einsatz von Quercetine als Quercetin- haltige Extrakte ebenfalls geeignet, bevorzugt dabei werden Dihydroquercetin-haltige Extrakte eingesetzt.

Die Extrakte von Quercetinen können mit Wasser, sowie polaren oder unpolaren organischen Lösungsmitteln sowie Mischungen davon in dem Fachmann bekannter Weise hergestellt werden. Extrakte, die durch Extraktion mit Ethanol oder Wasser/Ethanol-Mischungen, erhalten werden können, sowie Presssaft, sind bevorzugt.

Es können sowohl die Extrakte im ursprünglichen Extraktionsmittel als auch Extrakte/Presssaft in Wasser oder anderen organischen Lösungsmitteln und/oder deren Gemisch, insbesondere Ethanol sowie Ethanol/Wasser-Mischungen eingesetzt werden. Bevorzugt wird extrahiertes oder gepresstes Material als Feststoff eingesetzt, dem das Lösungsmittel (insbesondere möglichst schonend) entzogen wurde. Es können aber auch solche Extrakte/Presssäfte eingesetzt werden, denen das Lösungsmittel zum Teil entzogen wurde, so dass ein verdickter Extrakt/Presssaft eingesetzt wird.

Vorzugsweise wird ein Extrakt der Firma Bioflavon Ltd. namens Flavocon eingesetzt. Dieser Extrakt der Sibirischen Lerche enthält ca. 98 % Flavonoide, wobei davon ca. 90 % aus Dihydroquercetin bestehen.

Es konnte gezeigt werden, dass die ATP-Menge in den behandelten Follikeln signifikant im Vergleich zur einer Placeboformulierung erhöht wird. Damit wird dem biologisch aktiven Teil des Haares signifikant mehr ATP als Energielieferant für biochemische Synthesen und Transportvorgänge zur Verfügung gestellt, so dass Stoffwechselvorgänge und zelluläre Strukturen optimal aufrecht erhalten und erneuert werden können (Beispiel 7). Das Haar wird dadurch gekräftigt und vitalisiert und kann Schädigungen besser reparieren bzw. neues Haar aufbauen. Die Steigerung der Stoffwechselaktivität begünstigt das Haarwachstum, da für die zu Grunde liegenden Prozesse ausreichend Bausteine wie z.B. Aminosäuren zum Proteinaufbau bereitgestellt werden müssen; die dafür benötigte Energie wird z.B. durch die Verstoffwechselung von Glucose bereitgestellt.

Weiterhin zeigten Untersuchungen an dermalen Papillenzellen überraschenderweise, dass der Einsatz der erfindungsgemäßen Mittel zu einer Anregung des Haarwachstums und einer Verminderung vorzeitigen Haarausfalls führt. Es konnte beispielsweise die Ausschüttung des Wachstumsfaktors HGF (Hepatozytenwachstumsfaktor, Hepatocyte Growth Factor) und KGF (Keratinozytenwachtumsfaktor, Keratinocyte Growth Factor) in dermalen Papillenzellen nachgewiesen werden.
Ebenfalls wird die Aktivität des Gens für IGFBP3 (Insulin-like Growth Factor Binding Protein 3) verringert.
Zusätzlich konnte die signifikante Erhöhung der Aktivität des Gens für HGF und KGF nachgewiesen werden. (Beispiele 8, 9 und 10)

Ein weiterer Vorteil der vorliegenden Erfindung ist, dass die erfindungsgemäßen Mittel in der Lage sind, die Haarstruktur positiv zu beeinflussen, in dem die Haarspezifischen Strukturproteine (die Haarkeratine) stimuliert werden. So konnte überraschenderweise gezeigt werden, dass die Genexpression der Haarkeratine, hHa3-I und hHa4 signifikant erhöht wird. Dadurch wird die Haarstruktur, und somit das Haar, gekräftigt und gestärkt. Durch die Beeinflussung der Haarstruktur schon in den Haarwurzeln kann das Haar kräftig und gesund nachwachsen, ohne Nebenerscheinungen wie z.B. die Akkumulation von Pflegesubstanzen auf der Haarfaser (Beispiel 11).

Ein besonderer Einfluss der erfindungsgemäßen Mittel konnte auf die biologische Haarverdickung festgestellt werden. Die Anregung der Keratinozyten, die für die Bildung des Haarschaftes verantwortlich sind, erfolgt über die Wachstumsfaktoren HGF und KGF. Durch die Haarverdickung auf biologischer Basis werden Effekte wie die "Überpflege" der Haare vermieden. Das Haar wächst von der Wurzel an gestärkt und mit einem größeren Durchmesser nach, so dass dieser Effekt besonders langanhaltend ist. Dieser Effekt konnte in vivo ebenfalls nachgewiesen werden (Beispiel 12).

Das erfindungsgemäße Mittel enthält vorzugsweise 0,00001 bis 5 Gew.-%, vorzugsweise 0,0001 bis 2,5 Gew.-%, besonders bevorzugt 0,0001 bis 1 Gew.-% und insbesondere 0,00025 bis 0,5 Gew.-% Dihydroquercetin oder Dihydroquercetin-haltige Extrakte, bezogen auf das gesamte Mittel.

Die erfindungsgemäßen Mittel können weiterhin Hilfs- und Zusatzstoffe enthalten, die üblicherweise den jeweiligen kosmetischen Mitteln zugesetzt werden.

Demgemäß können die erfindungsgemäßen Mittel beispielsweise zusätzliche Bestandteile enthalten, ausgewählt aus der Gruppe, umfassend Proteinhydrolysate, filmbildende Substanzen, weitere haarwuchsstimulierende Wirkstoffe, nichtionogene Tenside, anionische Tenside, zwitterionische Tenside, ampholytische Tenside, nichtionische Polymere, Pflegestoffe, Verdickungsmittel, Parfümöle, Farbstoffe, Lichtschutzmittel, Antioxidantien, Antischuppenwirkstoffe, Treibmittel, Reduktionsmittel.
Weiterhin können die erfindungsgemäßen Mittel ein kationisches Tensid ausgewählt aus quartären Ammoniumverbindungen, Esterquats und Amidoaminen enthalten.

Bei den erfindungsgemäßen Mitteln handelt es sich vorzugsweise um Shampoos, Haarnachspülmittel, Haarwässer, Haargele, Haarkuren, Haarcreme, Haarlotionen, Haarsprays oder Haartinkturen.

Nach der Applikation des erfindungsgemäßen Mittels in Form von Haargel, Haarwasser, Haarkur, Haarcreme, Haarlotion, Haarspray und Haartinktur ist es zumeist nicht notwendig das Haar bzw. die Kopfhaut noch einmal auszuspülen oder mit weiteren zusätzlichen Mitteln zu behandeln, um eine positive Wirkung des Mittels zu erreichen. Die Mittel können natürlich ausgespült werden, vorzugsweise sollten sie jedoch auf dem Haar verbleiben.
Bei Shampoos und Haarnachspülmitteln kann das Mittel nach einer Einwirkzeit ausgespült werden. Dieses Ausspülen kann mit reinem Wasser oder einem marktüblichen Shampoo erfolgen. Einwirkzeiten von 10 Sekunden bis 15 Minuten haben sich in den meisten Fällen als ausreichend erwiesen.
Unabhängig von dem genauen Ablauf der Behandlung hat es sich als vorteilhaft erwiesen, die Mitteln bei einer Temperatur von 20 bis 55°C, insbesondere von 35 bis 40°C, anzuwenden.

Erfindungsgemäß von besonderem Interesse sind Haartonics, insbesondere als auf dem Haar verbleibende (leave on) Formulierung. Diese werden vorzugsweise bei Raumtemperatur angewendet, der alkoholische Gehalt liegt bevorzugtermaßen im Bereich von etwa 30 % bis etwa 35 % und der pH-Wert sollte etwa bei pH 7 liegen.

Als Pflegestoff kann beispielsweise ein Silikonöl und/oder ein Silikongum eingesetzt werden. In einer besonderen Ausführungsform der Erfindung enthalten die Mittel mindestens ein Silikonöl und/oder ein Silikongum.
Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Obwohl das erfindungsgemäße Mittel als Pflegestoff vorzugsweise ein Silikonderivat enthält, ist es auch möglich, dass das Mittel statt oder neben einer Silikonkomponente mindestens einen Pflegestoff einer anderen Verbindungsklasse enthält.

Als Pflegestoff einer anderen Verbindungsklasse kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Besonders interessant ist der Einsatz von Seiden-Proteinhydrolysaten. Unter Seide versteht man die Fasern des Kokons des Maulbeer-Seidenspinners (Bombyx mori L.). Die Rohseidenfaser besteht aus einem Doppelfaden Fibroin. Als Kittsubstanz hält Sericin diesen Doppelfaden zusammen. Seide besteht zu 70 - 80 Gew.-% aus Fibroin, 19 - 28 Gew.-% Sericin, 0,5 - 1 Gew.-% aus Fett und 0,5 - 1 Gew.-% aus Farbstoffen und mineralischen Bestandteilen.

Die wesentlichen Bestandteile des Sericin sind mit ca. 46 Gew.% Hydroxyaminosäuren. Das Sericin besteht aus einer Gruppe von 5 bis 6 Proteinen. Die wesentlichen Aminosäuren des Sericines sind Serin (Ser, 37 Gew.-%), Aspartat (Asp, 26 Gew.-%), Glycin (Gly, 17 Gew.-%), Alanin (Ala), Leucin (Leu) und Tyrosin (Tyr).

Das wasserunlösliche Fibroin ist zu den Skleroproteinen mit langkettiger Molekülstruktur zu zählen. Die Hauptbestandteile des Fibroin sind Glycin (44 Gew.-%), Alanin (26 Gew.-%), und Tyrosin (13 Gew.-%). Ein weiteres wesentliches Strukturmerkmal des Fibroins ist die Hexapeptidsequenz Ser-Gly-Ala-Gly-Ala-Gly.

Technisch ist es auf einfache Art und Weise möglich, die beiden Seidenproteine voneinander zu trennen. So verwundert es nicht, dass sowohl Sericin als auch Fibroin als Rohstoffe zur Verwendung in kosmetischen Produkten jeweils für sich allein bekannt sind. Weiterhin sind Proteinhydrolysate und -derivate auf der Basis der jeweils einzelnen Seidenproteine bekannte Rohstoffe in kosmetischen Mitteln. So wird beispielsweise Sericin als solches seitens der Fa. Pentapharm Ltd. als Handelsprodukt mit der Bezeichnung Sericin Code 303-02 vertrieben. Weitaus häufiger noch wird Fibroin als Proteinhydrolysat mit unterschiedlichen Molekulargewichten im Markt angeboten. Diese Hydrolysate werden insbesondere als "Seidenhydroylsate" vertrieben. So wird beispielsweise unter der Handelsbezeichnung Promois^{®} Silk hydrolysiertes Fibroin mit mittleren Molekulargewichten zwischen 350 und 1000 vertrieben.

Die positiven Eigenschaften der Seidenproteinderivate aus Sericin und Fibroin sind jeweils für sich genommen in der Literatur bekannt. So beschreibt die Verkaufsbroschüre der Fa. Pentapharm die kosmetischen Effekte des Sericines auf der Haut als reizlindernd, hydratisierend und filmbildend. Die Wirkung eines Fibroinderivates wird beispielsweise in der DE 31 39 438 A1 als pflegend und avivierend für das Haar beschrieben. Gemäß DE 102 40 757 A1 lässt sich bei einer gleichzeitigen Verwendung von Sericin und Fibroin bzw. deren Derivaten und/oder Hydrolysaten darüber hinaus eine synergistische Steigerung der positiven Wirkungen der Seidenproteine und deren Derivate erzielen.

Bevorzugt wird daher im erfindungsgemäßen Mittel als Seiden-Proteinhydrolysat ein Wirkstoffkomplex (A) bestehend aus dem Wirkstoff (A1) ausgewählt aus Sericin, Sericinhydrolysaten und/oder deren Derivaten, sowie Mischungen hieraus, und einem Wirkstoff (A2) ausgewählt aus Fibroin, und/oder Fibroinhydrolysaten und/oder deren Derivaten und/oder Mischungen hieraus eingesetzt.

Der Wirkstoffkomplex (A) verbessert signifikant in synergistischer Weise die zuvor dargestellten wesentlichen inneren und äußeren Strukturmerkmale und die Festigkeit sowie die Elastizität von menschlichen Haaren.

Als Wirkstoffe (A1) können im Wirkstoffkomplex (A) verwendet werden:
- natives Sericin,
- hydrolysiertes und/oder weiter derivatisiertes Sericin, wie beispielsweise Handelsprodukte mit den INCI - Bezeichnungen Sericin, Hydrolyzed Sericin, oder Hydrolyzed Silk,
- eine Mischung aus den Aminosäuren Serin, Aspartat und Glycin und/oder deren Methyl-, Propyl-, iso-Propyl-, Butyl-, iso-Butylestern, deren Salze wie beispielsweise Hydrochloride, Sulfate, Acetate, Citrate, Tartrate , wobei in dieser Mischung das Serin und/oder dessen Derivate zu 20 bis 60 Gew.%, das Aspartat und/oder dessen Derivate zu 10 - 40 Gew.% und das Glycin und/oder dessen Derivate zu 5 bis 30 Gew.% enthalten sind, mit der Maßgabe, dass sich die Mengen dieser Aminosäuren und/oder deren Derivate vorzugsweise zu 100 Gew.% ergänzen,
- sowie deren Mischungen.

Als Wirkstoffe (A2) können im Wirkstoffkomplex (A) verwendet werden:
- natives, in eine lösliche Form überführtes Fibroin,
- hydrolysiertes und/oder weiter derivatisiertes Fibroin, besonders teilhydrolisiertes Fibroin, welches als Hauptbestandteil die Aminosäuresequenz Ser-Gly-Ala-Gly-Ala-Gly enthält,
- die Aminosäuresequenz Ser-Gly-Ala-Gly-Ala-Gly,
- eine Mischung der Aminosäuren Glycin, Alanin und Tyrosin und/oder deren Methyl-, Propyl-, iso-Propyl-, Butyl-, iso-Butylestern, deren Salze wie beispielsweise Hydrochloride, Sulfate, Acetate, Citrate, Tartrate , wobei in dieser Mischung das Glycin und/oder dessen Derivate in Mengen von 20 - 60 Gew.%, das Alanin und dessen Derivate in Mengen von 10 - 40 Gew,% und das Tyrosin und dessen Derivate in Mengen von 0 bis 25 Gew.% enthalten sind, mit der Maßgabe, dass sich die Mengen dieser Aminosäuren und/oder deren Derivate vorzugsweise zu 100 Gew.-% ergänzen,
- sowie deren Mischungen.

Besonders gute pflegende Eigenschaften können erzielt werden, wenn eine der beiden Wirkstoffkomponenten des Wirkstoffkomplexes (A) in der nativen oder allenfalls löslich gemachten Form verwendet wird. Es ist auch möglich, eine Mischung aus mehreren Wirkstoffen (A1) und/oder (A2) einzusetzen.

Es kann bevorzugt sein, dass die beiden Wirkstoffe (A1) und (A2) im Verhältnis von 10:90 bis 70:30, insbesondere 15:85 bis 50:50 und ganz besonders 20:80 bis 40:60, bezogen auf deren jeweilige Gehalte an aktiver Wirksubstanz in den erfindungsgemäßen Mitteln eingesetzt werden.

Die Derivate der Hydrolysate von Sericin und Fibroin umfassen sowohl anionische als auch kationisierte Proteinhydrolysate. Die Proteinhydrolysate von Sericin und Fibroin sowie die daraus hergestellten Derivate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche Proteinhydrolysate von Sericin und Fibroin und/oder deren Derivate, deren zugrunde liegender Proteinanteil ein Molekulargewicht von 100 bis zu 25000 Dalton, bevorzugt 250 bis 10000 Dalton aufweist. Weiterhin sind unter kationischen Proteinhydrolysaten von Sericin und Fibroin auch quaternierte Aminosäuren und deren Gemische zu verstehen. Die Quaternisierung der Proteinhydrolysate oder der Aminosäuren wird häufig mittels quartärer Ammoniumsalze wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäß einsetzbaren kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Silk, Cocodimonium Hydroxypropyl Silk Amino Acids, Hydroxyproypltrimonium Hydrolyzed Silk, Lauryldimonium Hydroxypropyl Hydrolyzed Silk, Steardimonium Hydroxypropyl Hydrolyzed Silk, Quaternium-79 Hydrolyzed Silk. Als typische Beispiele für die erfindungsgemäßen anionischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Potassium Cocoyl Hydrolyzed Silk, Sodium Lauroyl Hydrolyzed Silk oder Sodium Stearoyl Hydrolyzed Silk. Letztlich seien noch als typische Beispiele für die erfindungsgemäß einsetzbaren Derivate aus Sericin und Fibroin die unter den INCI - Bezeichnungen im Handel erhältlichen Produkte genannt: Ethyl Ester of Hydrolyzed Silk und Hydrolyzed Silk PG-Propyl Methylsilanediol. Weiterhin erfindungsgemäß verwendbar, wenngleich nicht unbedingt bevorzugt sind die im Handel erhältlichen Produkte mit den INCI - Bezeichnungen Palmitoyl Oligopeptide, Palmitoyl Pentapeptide-3, Palmitoyl Pentapeptide-2, Acetyl Hexapeptide-1, Acetyl Hexapeptide-3, Copper Tripeptide-1, Hexapeptide-1, Hexapeptide-2, MEA-Hydrolyzed Silk.

Die Wirkung des Wirkstoffkomplexes (A) kann durch die Zugabe von Fettstoffen weiter gesteigert werden. Unter Fettstoffen sind zu verstehen Fettsäuren, Fettalkohole, natürliche und synthetische Wachse, welche sowohl in fester Form als auch flüssig in wässriger Dispersion vorliegen können, und natürliche und synthetische kosmetische Ölkomponenten zu verstehen.

Proteinhydrolysate pflanzlichen Ursprungs, z. B. Soja-, Mandel-, Erbsen-, Kartoffel- und Weizenproteinhydrolysate, sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Croiastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als filmbildende Substanzen können einige pflegende Polymere auch filmbildende und/oder festigende Eigenschaften aufweisen, und daher auch als filmbildende und/oder festigende Polymere eingesetzt werden können.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 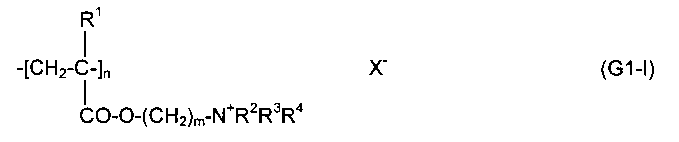 in der R¹= -H oder -CH₃ ist, R², R³ und R⁴ unabhängig voneinander ausgewählt sind aus C₁-₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliche Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quartären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silikon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäß einsetzbare kationische Polymere sind die so genannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugt eingesetzte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Bevorzugt eingesetzte amphotere Polymere sind solche Polymerisate, die sich im wesentlichen zusammensetzen aus
(a) Monomeren mit quartären Ammoniumgruppen der allgemeinen Formel (II),

   R¹-CH=CR²-CO-Z-(CₙH₂ₙ)-N⁽⁺⁾R³R⁴R⁵ A⁽⁻⁾ (II)

   in der R¹ und R² unabhängig voneinander stehen für Wasserstoff oder eine Methylgruppe und R³, R⁴ und R⁵ jeweils unabhängig voneinander für eine Alkylgruppe mit 1 bis 4 Kohlenstoffatomen, Z eine NH-Gruppe oder ein Sauerstoffatom, n eine ganze Zahl von 2 bis 5 und A das Anion einer organischen oder anorganischen Säure ist, und
(b) monomeren Carbonsäuren der allgemeinen Formel (III),

   R⁶-CH=CR⁷-COOH (III)

   in denen R⁶ und R⁷ unabhängig voneinander für Wasserstoff oder eine Methylgruppe stehen.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-lon ist; Acrylamidopropyltrimethyl-ammoniumchlorid ist ein besonders bevorzugtes Monomeres (a). Als Monomeres (b) für die genannten Polymerisate wird bevorzugt Acrylsäure verwendet.

Die erfindungsgemäßen Mittel enthalten die pflegenden, kationischen und/oder amphoteren Polymere in bevorzugter Weise in einer Menge von 0,01 bis 5 Gew.-%, insbesondere in einer Menge von 0,1 bis 2 Gew.-%, jeweils bezogen auf die gesamte Anwendungszubereitung.

In einer besonders bevorzugten Ausführungsform enthalten die erfindungsgemäßen Mittel weitere haarwuchsstimulierende Wirkstoffe.

Geeignet sind alle bekannten haarwuchsstimuliernden Wirkstoffe, beispielsweise Minoxidil (6-Piperidino-2,4-pyrimidindiamin-3-oxid), Aminexil (Diaminopyrimidinoxid), Finasterid (N-tert-Butyl-3-oxo-4-aza-5α-androst-1-en-17β-carboxamid), Carnitintartrat und 5-α-Reduktaseinhibitoren.

Als 5-α-Reduktaseinhibitoren sind insbesondere geeignet die funktionellen C₂-C₁₂-Carbonsäuren und deren physiologisch verträglichen Metallsalze, insbesondere 10-Hydroxydecansäure, 10-Hydroxydecensäure und ihren Derivate, Derivate von C₃-C₉-Polyolen, Phenolderivate, Pflanzenextrakte, Riechstoffe, Flavonoide, Isoflavonoide, 6,7-disubstituierte 2,2-Dialkylchromane oder -chromene, Aluminiumchlorohydrat, 2-Phenylethanol, Etidronsäure, 7-Acetyl-1,1,3,4,4,6-hexamethyltetralin, Tropolonderivate, Ester der Schwefelsäure mit alkoxylierten C₈-C₁₈-Fettalkoholen und deren physiologisch verträgliche Metallsalze, Ester der Phosphorsäure und der Triphosphorsäure mit ein- bis sechswertigen Hydroxyverbindungen, Kieselsäureester, aus marinen Organismen isolierbare mycosporin-ähnliche Aminosäuren (MAA) sowie quaternäre Siliconverbindungen. Unter Derivaten sind dabei insbesondere deren Salze, Ester und Amide zu verstehen.

Die erfindungsgemäßen Mittel können weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Mittel enthalten die Vitamin A-Komponente bevorzugt in Mengen von 0,05-1 Gew.-%, bezogen auf die gesamte Anwendungszubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf die gesamte Anwendungszubereitung, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs sind in den erfindungsgemäßen Mitteln bevorzugt in Mengen von 0,05 - 10 Gew.-%, bezogen auf die gesamte Anwendungszubereitung, enthalten. Mengen von 0,1 - 5 Gew.-% sind besonders bevorzugt.
- Vitamin B₆ (Pyridoxin sowie Pyridoxamin und Pyridoxal). Die genannten Verbindungen des Vitamin B₆-Typs sind in den erfindungsgemäßen Mitteln bevorzugt in Mengen von 0,01 - 5 Gew.-%, bezogen auf die gesamte Anwendungszubereitung, enthalten. Mengen von 0,05 - 1 Gew.-% sind besonders bevorzugt.

Vitamin C (Ascorbinsäure). Vitamin C wird in den erfindungsgemäßen Mitteln bevorzugt in Mengen von 0,1 bis 3 Gew.-%, bezogen auf die gesamte Anwendungszubereitung eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf die gesamte Anwendungszubereitung, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*,6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-%, jeweils bezogen auf die gesamte Anwendungszubereitung enthalten.

Bevorzugt enthalten die erfindungsgemäßen Mittel Vitamine, Provitamine und Vitaminvorstufen aus den Gruppen A, B, C, E und H.

Panthenol, Pantolacton, Pyridoxin und seine Derivate sowie Nicotinsäureamid und Biotin sind besonders bevorzugt.

Ganz besonders bevorzugt wird als Pflegestoff D-Panthenol, gegebenenfalls in Kombination mit mindestens einem der oben genannten Silikonderivate eingesetzt.

In einer bevorzugten Ausführungsform können die erfindungsgemäßen Mittel weiterhin mindestens einen Pflanzenextrakt enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng und Ingwerwurzel bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders geeignet sind die Extrakte aus Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 90 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.
Weiterhin können die erfindungsgemäßen als Pflegestoff eine Reihe von Carbonsäuren enthalten.

Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäß einsetzbaren Carbonsäuren können selbstverständlich entlang der Kohlenstoffkette oder des Ringgerüstes substituiert sein. Zu den Substituenten der erfindungsgemäß einsetzbaren Carbonsäuren sind beispielsweise zu zählen C₁-C₈-Alkyl-, C₂-C₈-Alkenyl-, Aryl-, Aralkyl- und Aralkenyl-, Hydroxymethyl-, C₂-C₈-Hydroxyalkyl-,C₂-C₈-Hydroxyalkenyl-, Aminomethyl-, C₂-C₈-Aminoalkyl-, Cyano-, Formyl-, Oxo-, Thioxo-, Hydroxy-, Mercapto-, Amino-, Carboxy- oder Iminogruppen. Bevorzugte Substituenten sind C₁-C₈-Alkyl-, Hydroxymethyl-, Hydroxy-, Amino- und Carboxygruppen. Besonders bevorzugt sind Substituenten in α-Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Als Beispiele für erfindungsgemäß einsetzbare Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I), 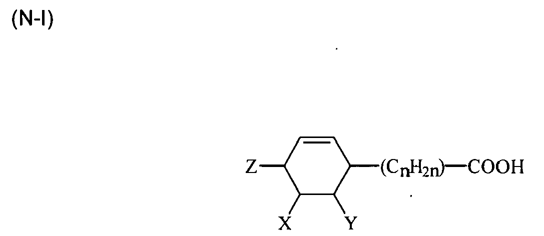 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Dicarbonsäuren der Formel (N-I) sind in der Literatur bekannt. So ist beispielweise US-A 3,753,968 ein Herstellungsverfahren zu entnehmen.

Die Dicarbonsäuren der Formel (N-I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuss vorliegt. Diese Isomerengemische können erfindungsgemäß ebenso wie die reinen Verbindungen eingesetzt werden.

Erfindungsgemäß einsetzbar neben den bevorzugten Dicarbonsäuren gemäß Formel (N-I) sind auch solche Dicarbonsäuren, die sich von den Verbindungen gemäß Formel (N-I) durch 1 bis 3 Methyl- oder Ethyl-Substituenten am Cyclohexylring unterscheiden oder aus diesen Verbindungen formal durch Anlagerung von einem Molekül Wasser an die Doppelbindung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid^{®} 1550 und Westvaco Diacid^{®} 1595 (Hersteller: Westvaco) erhältlich.

Neben den zuvor beispielhaft aufgeführten kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt 2-Pyrrolidinon-5-carbonsäure und deren Derivate als Carbonsäure einzusetzen. Besonders bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf die gesamte Anwendungszubereitung, besonders bevorzugt 0,1 bis 5 Gew.%, und insbesondere bevorzugt 0,1 bis 3 Gew. %.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren einzusetzen. Hierbei hat sich gezeigt, dass neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C₁₂-C₁₅-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

Weiterhin können die erfindungsgemäßen Mittel als Pflegestoff Ölkörper enthalten.

Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I), 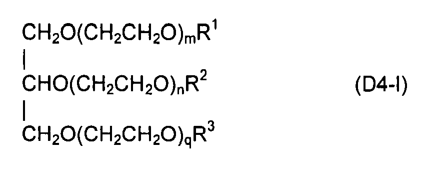 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Das Mittel kann überdies ein Enzym als Pflegestoff enthalten. Erfindungsgemäß besonders bevorzugte Enzyme sind ausgewählt aus einer Gruppe, die gebildet wird aus Proteasen, Lipasen, Transglutaminase, Oxidasen und Peroxidasen.

In Abhängigkeit von der Art des erfindungsgemäßen Mittels kann es erforderlich sein, dass diese weiterhin mindestens ein Tensid enthalten. Dies gilt insbesondere für Shampoos. Aber auch andere Mittel, wie beispielsweise Haarspülungen, Haarkuren, Haarwasser, Haarcreme, Haarlotionen, Haartinkturen.

Beispielsweise können kationische Tenside eingesetzt werden.
Erfindungsgemäß bevorzugt sind kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäßen Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Neben oder statt der kationischen Tenside können die Mittel weitere Tenside oder Emulgatoren enthalten, wobei prinzipiell sowohl anionische als auch ampholytische und nichtionische Tenside und alle Arten bekannter Emulgatoren geeignet sind. Die Gruppe der ampholytischen oder auch amphoteren Tenside umfasst zwitterionische Tenside und Ampholyte. Die Tenside können bereits emulgierende Wirkung haben.

In einer bevorzugten Ausführungsform der vorliegenden Erfindung wird, beispielsweise in einem Shampoo, eine Kombination aus anionischen und nichtionischen Tensiden oder eine Kombination aus anionischen und amphoteren Tensiden eingesetzt. In einem Haartonic kann der Fachmann jedoch auch weitgehend oder vollständig auf den Einsatz von Tensiden verzichten.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-l), 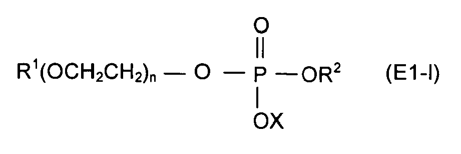 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4 - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Monoglyceridsulfate und Monoglyceridethersulfate der Formel (E1-III) 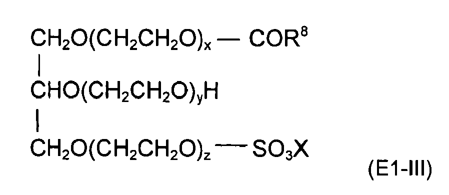 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine -COO⁽⁻⁾ - oder-SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.
   Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III), 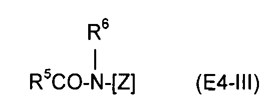 in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R⁷CO-NR⁸-CH₂-(CHOH)₄CH₂OH (E4-IV)

   Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. technischer Mischungen dieser Säuren steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können als nichtionische Tenside die Zuckertenside enthalten sein. Diese sind bevorzugt in Mengen von 0,1 bis 20 Gew.-%, bezogen auf die jeweilige gesamte Zusammensetzung, enthalten. Mengen von 0,5 bis 15 Gew.-% sind besonders bevorzugt, und ganz besonders bevorzugt sind Mengen von 0,5 bis 7,5 Gew.-%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Die weiteren Tenside werden in der Regel in Mengen von 0,1 bis 45 Gew.-%, bevorzugt 0,5 bis 30 Gew.-% und ganz besonders bevorzugt von 0,5 bis 25 Gew.-%, bezogen auf die jeweilige gesamte Zusammensetzung, eingesetzt. Dabei hängt die eingesetzte Menge wesentlich davon ab, welchen Zweck das erfindungsgemäße Mittel erfüllt. Handelt es sich um ein Shampoo oder ein anderes reinigendes Mittel, sind auch Tensidmengen über 45 Gew.% üblich.

Gemäß einer weiteren, bevorzugten Ausführungsform der Erfindung enthalten die Mittel weiterhin mindestens einen Emulgator und/oder mindestens einen Dialkylether. Durch den Zusatz einer oder mehrerer dieser Stoffe wird die Verfügbarkeit der Wirkstoffe in der Haarwurzel positiv beeinflusst. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 100 Mol Ethylenoxid und/oder 1 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- lineare und verzweigte Fettsäuren mit 8 bis 30 C - Atomen und deren Na-, K-, Ammonium-, Ca-, Mg- und Zn - Salze.

Die Emulgatoren werden bevorzugt in Mengen von 0,1 bis 25 Gew.-%, insbesondere 0,1 bis 3 Gew.-%, bezogen auf die jeweilige gesamte Zusammensetzung, eingesetzt.

Erfindungsgemäß besonders geeignete Emulgatoren sind Polyethylenglycolalkylether mit Alkylkettenlängen von 6 bis 30 C-Atomen, bevorzugt von 12 bis 22 C-Atomen und einem Ethoxylierungsgrad von 1 bis 1000, bevorzugt von 1 bis 500 und insbesondere von 1 bis 100.

Insbesondere bevorzugte Polyethylenglycolether im Sinne der Erfindung sind die unter der INCI-Bezeichnung bekannten Stoffe Ceteareth-6, Ceteareth-9, Ceteareth-10, Ceteareth-20, Ceteareth-30, Ceteareth-25, Steareth-10 und Steareth-20.

Weiterhin besonders geeignete Emulgatoren im Sinne der Erfindung sind C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin. Insbesondere geeignet sind PEG-20 Glyceryl Stearate, PEG-7 Glyceryl Cocoate, PEG-30 Glyceryl Stearate, PEG-6 Caprylic/Capric Glycerides, Polyglyceryl-3-diisostearate und Glycereth-2-Cocoate.

Bevorzugt sind nichtionogene Emulgatoren mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen. Nichtionogene Emulgatoren mit einem HLB-Wert von 10 bis 16 sind erfindungsgemäß besonders bevorzugt.

Erfindungsgemäß geeignete Dialkylether sind Di-n-alkylether mit insgesamt 5 bis 50 C-Atomen, bevorzugt mit 8 und 40 und insbesondere mit 12 bis 24 C-Atomen, wie beispielsweise Di-n-octylether (Cetiol^{®}OE), Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether, 2-Methyl-pentyl-n-octylether und 1,3-Di-(2-ethyl-hexyl)-cyclohexan (Cetiol^{®} S).
Erfindungsgemäß besonders geeignet ist Dicaprylylether.

Die erfindungsgemäßen Mittel enthalten die Dialkylether bevorzugt in Mengen von 0,01 - 50 Gew.-%, bevorzugt in Mengen von 0,05 bis 20 Gew.-% und insbesondere in Mengen von 0,1 - 15 Gew.-%, bezogen auf das gesamte Mittel.

Es kann weiterhin erforderlich sein, dass die erfindungsgemäßen Mittel weiterhin mindestens ein Verdickungsmittel enthalten. Dies trifft insbesondere dann zu, wenn es sich bei dem Mittel um ein Gel oder eine Paste handelt.

Geeignete Verdickungsmittel sind beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit, vollsynthetische Hydrokolloide wie z.B. Polyvinylalkohol, und gegebenenfalls vernetzte Polyacrylate. Auch die Polymere, die als mögliche Pflegestoffe beschrieben wurden oder im Folgenden als filmbildende und/oder festigende Polymere aufgezählt werden, können verdickende Eigenschaften aufweisen, und können gegebenenfalls als Verdickungsmittel Verwendung finden.

Die Mittel können neben den genannten Komponenten weiterhin alle für entsprechende kosmetische Mittel bekannten Wirk-, Zusatz- und Hilfsstoffe enthalten. Dies sind beispielsweise
- Strukturanten wie Maleinsäure und Milchsäure,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Substanzen zur Einstellung des pH-Wertes, wie beispielsweise übliche Säuren, insbesondere Genusssäuren, und Basen,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie der eingesetzten Mengen dieser Komponenten wird ausdrücklich auf die dem Fachmann bekannten einschlägigen Handbücher verwiesen.

Die Formulierung der erfindungsgemäßen Mittel kann in allen für kosmetische Mittel üblichen Formen erfolgen, beispielsweise in Form von Lösungen, die als Haarwasser oder Pump- oder Aerosolspray auf das Haar aufgebracht werden können, in Form von Emulsionen, Wachsen, Gelen oder auch tensidhaitigen schäumenden Lösungen oder anderen Zubereitungen, die für die Anwendung auf dem Haar geeignet sind.

In einer bevorzugten Ausführungsform handelt es sich bei den erfindungsgemäßen Mitteln um Haarbehandlungsmittel, insbesondere Shampoo, Haarnachspülmittel, Haartonic, Haargel, Haarwasser, Haarkur, Haarcreme, Haarlotion, Haarspray und Haartinktur.

Die erfindungsgemäßen Mittel können beispielsweise als Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, Gele, Sprays, Aerosole und Schaumaerosole formuliert vorliegen. Die Mittel können als Einkammersystem oder als Zweikammersystem konfektioniert werden.

Sofern es sich bei den erfindungsgemäßen Mitteln um ein Aerosolprodukt handelt, enthält dieses zwingend ein Treibmittel.

Erfindungsgemäß geeignete Treibmittel sind beispielsweise N20, Dimethylether, CO2, Luft und Alkane mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und deren Mischungen.

Bevorzugt werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Wenngleich die zuvor genannten Haarbehandlungsmittel erfindungsgemäß bevorzugt sind, kann es sich bei den erfindungsgemäßen Mitteln auch um andere Haarbehandlungsmittel, wie z.B. Haarfärbemittel und Wellmittel, handeln. Diese Mittel enthalten dann gegebenenfalls die bekannten direktziehenden Farbstoffe, Vorläufer für Oxidationsfarbstoffe (Entwickler- und Kupplerkomponenten) und Oxidationsmittel bzw. Reduktionsmittel. Vorteilhafterweise können die erfindungsgemäßen Mittel so das Haar vor der Beanspruchung bei der Färbung schützen, das Haarfollikel aktivieren und gleichzeitig Hautirritationen durch die Well- bzw. Haarfärbemitteln vermindern bzw. lindern.

Ein weiterer Gegenstand der Erfindung ist die Verwendung der erfindungsgemäßen Mittel zur Vitalisierung von Haaren, Reaktivierung der Haarwurzel, Aktivierung von Haarfollikeln, Haarverdickung, Förderung oder Verstärkung des Haarwachstums, zur Haarkonditionierung, zur Verminderung von Haarausfall und/oder zur Behandlung von Haarausfall.

Ein weiterer Gegenstand der vorliegenden Erfindung ist ein kosmetisches Verfahren zur Vitalisierung von Haaren, Reaktivierung der Haarwurzel, Aktivierung von Haarfollikeln, Förderung oder Verstärkung des Haarwachstums, zur Haarkonditionierung, zur Verminderung von Haarausfall und / oder zur Behandlung von Haarausfall, wobei die erfindungsgemäßen Mittel auf die Haare, Kopfhaut, behaarte Haut bzw. auf Hautstellen, wo der Haarwuchs seit längerem eingestellt ist, wo jedoch ein Haarwuchs gewünscht wird, aufgebracht wird.

Die nachfolgenden Beispiele sollen den Gegenstand der vorliegenden Erfindung erläutern ohne ihn in irgendeiner Weise zu beschränken.

### Informationen zu den in den Beispielen eingesetzten Stoffen:

### Dihydroquercetin (3,3',4',5,7-Pentahydroxyflavanone, Taxifolin, 2-(3.4-dihyrdroxy-phenyl)-3 5 7-trihydroxy-chroman-4-one)

Verwendet wurde in den beschriebenen Beispielen ein Extrakt der Firma Bioflavon Ltd. namens Flavocon. Dieser Extrakt der Sibirischen Lerche enthält ca. 98 % Flavonoide, wobei davon ca. 90 % aus Dihydroquercetin (DHQ) bestehen. Daraus leitet sich ab, dass der Extrakt zu ca. 88 % aus Dihydroquercitin besteht. 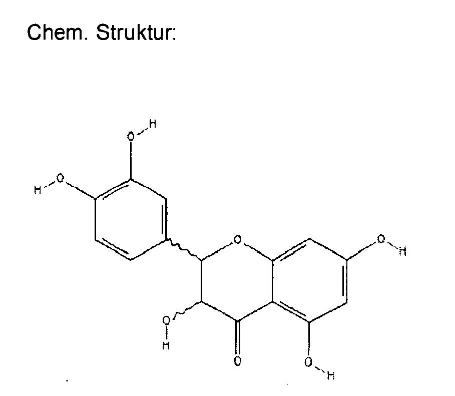
Carbopol ETD2020
   Noveon (Quimidroga)
   Acrylic acid copolymer
   ACRYLATES/C10-30 ALKYL ACRYLATE CROSSPOLYMER
Proteinextrakt aus Soja
   Cosmetochem
   PROPYLENE GLYCOL, AQUA , GLYCINE SOJA SEED EXTRACT
Tee-Extrakt
   Cosmetochem
   CAMELLIA SINENSIS LEAF EXTRACT
Octopirox
   Clariant
   Hydroxy-4-methyl-6(2,4,4-trimethylpentyl)-2-pyridone-monoethanolamine salt
   PIROCTONE OLAMINE
Rovisome CT
   Rovi
   pH 5.5-6.5 MD 200-500nm
   AQUA (WATER), ALCOHOL DENAT., LECITHIN, CARNITINE TARTRATE
Cremophor A25
   Cremophor O (formerly)
   BASF
   Fatty alcohols, C16-18, ethoxylated (25 EO)
CETEARETH-25
FAEOS-Na C12-14 2 EO 70%
   Cognis Deutschland GmbH
   SODIUM LAURETH SULFATE
Antil 141 L
   Goldschmidt (Degussa)
   Polyethoxypropylene glycoldioleate
   PROPYLENE GLYCOL, PEG-55 PROPYLENE GLYCOL OLEATE
C*Pharm 02010
   Cerestar (Inter-Harz GmbH)
   Glucose H2_O *D(+)-
   Grape sugar H2_O
   GLUCOSE
PEG-40 hydrogenated Castor Oil 455
   Cognis Deutschland GmbH
   PEG-40 HYDOGENATED CASTOR OIL, PROPYLENE GLYCOL
Euperlan PK 3000 AM
   Cognis Deutschland GmbH
   AQUA (WATER), GLYCOL DISTEARATE, GLYCERIN, LAURETH-4, COCAMIDOPROPYL
   BETAINE, FORMIC ACID
Timiron Supersheen MP-1001
   Iriodin Ti 100 FK (formerly)
   Merck
   MICA, Cl 77891 (TITANIUM DIOXIDE)
Cutina CP
   Cutina CP V (formerly)
   Cutina CP PF (formerly)
   Cognis Deutschland GmbH
   Cetyl palmitate (vegetable base)
   CETYL PALMITATE
Eumulgin B2
   Disponil B 3
   Cognis Deutschland GmbH
   Fatty alcohols, C16-18, ethoxylated (30 EO)
   CETEARETH-30
Genamin KDMP
   Clariant
   Trimethyl-N(C20-22-alkyl)ammonium chloride *N,N,N- BEHENTRIMONIUM CHLORIDE
Structure XL (28-030A)
   National Starch
   HYDROXYPROPYL STARCH PHOSPHATE
Wacker-Belsil ADM 8020 VP
   Wacker
   AMODIMETHICONE, TRIDECETH-5, GLYCERIN, TRIDECETH-10
Gluadin WQ
   Cognis Deutschland GmbH,
   AQUA (WATER), LAURDIMONIUM HYDROXYPROPYL HYDROLYZED WHEAT PROTEIN, ETHYLPARABEN,METHYLPARABEN
   Protein hydrolyzates, wheat germ, (3-(dodecyldimethylammonio)-2-hydroxypropyl), chlorides ca. 30-35 % Gehalt
Gluadin WLM
   Cognis Deutschland GmbH
   Weizenproteinhydrolysat in H2O
   INCI declaration [INCI] HYDROLYZED WHEAT PROTEIN
   Gehalt ca. 20-24 %
Cetiol HE
   Cognis Deutschland GmbH
   Kokosmonoglycerid ethoxyliert (7 EO)
   INCI declaration [INCI] PEG-7 GLYCERYL COCOATE
Ajidew NL 50
   Ajinomoto
   Pyrrolidoncarbonsäure Natrium Salz
   Na-PCA
SODIUM
Arlypon F
   Cognis Deutschland GmbH
   Lauromacrogol JP 12 (Pharmacopoe of Japan)
   *NLP
   C12-14 Fettalkohole ethoxyliert (2.5 EO)
   LAURETH-2
Synthalen K
   Synthalen KD (alt)
   3V Sigma
   Polyacrylsäure
   CARBOMER
Neutrol TE
   BASF
   Tetrakis-(2-hydroxypropyl)-ethylendiamin *N,N,N',N',-Edetol
   TETRAHYDROXYPROPYL ETHYLENEDIAMINE

### Beispiele

### Beispiel 1: Haartonic

| | |
|---|---|
| Wasser | ad 100 |
| Carbopol ETD 2020 | 0,1 |
| D-Biotin | 0,01 |
| Allantoin | 0,5 |
| PEG-40 Hydrogenated Castor Oil | 0,15 |
| D-Panthenol | 0,1 |
| Ethanol vergällt | 40 |
| Proteinextrakt aus Soja | 0,3 |
| Glycerin | 0,2 |
| Extrakt Tee | 0,2 |
| Dihydroquercetin | 0,01 |

### Beispiel 2: Haartonic

| | |
|---|---|
| Wasser, vollentsalzt | ad 100 |
| D-Panthenol 75 % | 0,2 |
| Allantoin | 0,2 |
| Benzophenone-4 | 0,05 |
| Synthalen K | 0,3 |
| Neutrol TE | 0,25 |
| Ethanol 96 % DEP vergällt | 40 |
| Octopirox | 0,2 |
| Menthol, natürlich | 0,03 |
| Parfum Donna E-0103703 | 0,3 |
| Dihydroquercetin | 0,01 |

### Beispiel 3: Haartonic

| | |
|---|---|
| D-Panthenol 75 % | 0,2 |
| Allantoin | 0,1 |
| Wasser, vollentsalzt | ad 100 |
| Rovisome CT | 40 |
| Ethanol 96 % DEP vergällt | 35 |
| Cremophor A25 | 0,2 |
| Coffein | 0,1 |
| Dihydroquercetin | 0,01 |

### Beispiel 4: Shampoo

| | |
|---|---|
| FAEOS-Na C12-14 2EO 70% | 15,4 |
| Natronlauge 50% Standard | 0,2 |
| Stadtwasser U-K | ad 100 |
| Citronensäure Monohydrat Standard | 0,6 |
| Antil 141 L | 0,5 |
| Arlypon F | 0,6 |
| Glycin | 0,3 |
| C*Pharm 02010 | 1 |
| Coffein wasserfrei | 0,1 |
| alpha-Bisabolol, natuerlich | 0,1 |
| Magnesiumchlorid Schuppen | 0,02 |
| D-Panthenol 75 % | 0,4 |
| Nikotinsäureamid | 0,2 |
| Salicylsäure | 0,2 |
| Disodium Cocoamphodiacetate | 7,5 |
| Na-benzoat | 0, 5 |
| POLYQUATERNIUM 10 | 0,3 |
| Parfum | 0,7 |
| Cetiol HE | 1,5 |
| PEG-40 Hydrogenated Castor Oil 455 | 0,4 |
| Natriumchlorid fein-mittel | 0,1 |
| Dihydroquercetin | 0,1 |

### Beispiel 5: Pflegeshampoo

| | |
|---|---|
| FAEOS-Na C12-14 2EO 70% | 15,4 |
| Natronlauge 50% Standard | 0,15 |
| Stadtwasser U-K | ad 100 |
| Citronensäure Monohydrat Standard | 0,7 |
| Antil 141 L | 0,3 |
| Arlypon F | 0,2 |
| Gluadin WQ | 0,1 |
| Euperlan PK 3000 AM | 5 |
| Pantolacton, Schuppen | 0,2 |
| Timiron Supersheen MP-1001 | 0,14 |
| Salicylsäure | 0,2 |
| Disodium Cocoamphodiacetate | 8 |
| Na-benzoat | 0,5 |
| Gluadin WLM | 0,5 |
| Hydrogenated Castor Oil | 0,2 |
| Cetiol HE | 0,5 |
| Natriumchlorid fein-mittel | 0,3 |
| POLYQUATERNIUM 10 | 0,8 |
| Dihydroquercetin | 0,1 |

### Beispiel 6: Pflegespülung

| | |
|---|---|
| Cutina CP | 0,6 |
| Eumulgin B 2 | 0,4 |
| Cetearyl Alcohol | 5 |
| Genamin KDMP | 4 |
| Propylparaben | 0,2 |
| Benzophenone-4 | 0,4 |
| Structure XL (28-030A) | 0,8 |
| Stadtwasser U-K | ad 100 |
| Methylparaben | 0,2 |
| Phenoxyethanol, rein | 0,4 |
| Wacker-Belsil ADM 8020 VP | 2 |
| Ajidew NL 50 | 0,5 |
| Glycin | 0,4 |
| Dihydroquercetin | 0,2 |

### Beispiel 7: Bestimmung der ATP-Syntheserate

ATP (Adenosintriphosphat) ist die universelle Speicherform für chemische Energie in Zellen. Bei der Abspaltung der distalen Phosphatgruppe entsteht ADP und Pᵢ (anorganisches Phosphat). Diese Reaktion ist stark exergon, d.h. es wird Energie frei. Produziert wird ATP beim zellulären, oxidativen Abbau von Fetten, Kohlehydraten und Proteinen. Es dient als Energielieferant für biochemische Synthesen, für Transportvorgänge (aktiver Transport) und für mechanische Arbeit. Diese Vorgänge sind endergon, d.h. sie laufen nur unter Energiezufuhr ab. Um ihren Stoffwechsel optimal aufrecht zu erhalten, sind Zellen also auf eine ausreichende Versorgung mit ATP angewiesen. Auch dermale Papillenzellen benötigen beispielsweise zur Produktion von Wachstumsfaktoren und damit zur Steuerung des Haarzyklus ATP. Die Proliferation und Differenzierung von Haarschaftkeratinozyten ist ebenfalls an die ATP-Synthese gekoppelt, da für beide Vorgänge die Biosynthese spezifischer Proteine essentielle Voraussetzung ist. Kann durch ein Produkt die ATP-Syntheserate der haarrelevanten Zellen erhöht werden, so steht den Zellen mehr Energie zur Verfügung um Stoffwechselvorgänge und zelluläre Strukturen aufrecht zu erhalten, und um Strukturen zu erneuern, z.B. bei Reparaturprozessen oder dem Neuaufbau von Haaren.

### ATP-Nachweismethode

Die ATP-Bestimmungen erfolgten mit Hilfe des ATPLiteTM-M Assays (Packard). Das Testprinzip dieses Assays beruht darauf, dass die Luciferase von Photinus pyralis eine Reaktion katalysiert, bei der in Gegenwart von ATP D-Luciferin in Oxyluciferin umgewandelt wird. Bei dieser Reaktion wird grünes Licht emittiert, das mit einem Luminometer gemessen werden kann. Das emittierte Biolumineszenzlicht ist proportional zur Menge des vorhandenen ATP.
Die Bestimmung der ATP-Aktivität erfolgt in organotypischen Zellkulturen. Die Behandlung mit dem Substanzgemisch erfolgte über 24 Stunden gegen eine unbehandelte Kontrolle. Anschließend wurden die Zellen mit jeweils 100 µl/Kavität eines im Testkit enthaltenen Lysepuffer für 5 min auf einem Schüttler lysiert. Danach wurden die Zellen für weitere 5 min mit jeweils 100 µl/Kavität mit der mitgelieferten Substratlösung auf dem Schüttler inkubiert und anschließend das Reaktionsgemisch in eine schwarze Mikrotiterplatte überführt. Nach einer Inkubationszeit von 10 min in der Dunkelheit erfolgte die Messung der Lumineszenz.

**Tabelle 1: Einfluss von Dihydroquercetin auf die ATP-Produktion von organotypischen Zellkulturen in %**

| | Mittelwert (Standardabweichung) |
|---|---|
| Unbehandelte Probe | 100 (2) |
| Dihydroquercetin 88 µM | 138 (10) |
| (Entspricht Extrakt der Sibirischen Lerche von 0,003%) | |

Dihydroquercetin steigerte die ATP-Produktion der organotypischen Zellkulturen gegenüber der unbehandelten Kontrolle um 38% und unterstützt derart die Syntheseleistungen an der Haarwurzel.

### Beispiel 8: Nachweis der differentiellen Expression von haarrelevanten Genen

Hepatocyte Growth Factor (HGF) und Keratinocyte Growth Factor (KGF) sind wichtige Wachstumsfaktoren, die von den dermalen Papillenzellen synthetisiert werden. Im Speziellen stimuliert HGF das Follikelwachstum und KGF die Keratinozytenproliferation. Entsprechend werden HGF und KGF in der Wachstumsphase vermehrt exprimiert. Diesen Eigenschaften gegenüber steht die Expression von IGFBP-3. Dieses Genprodukt führen zu einer Verschiebung des Zyklus von der Wachstums- zur Regressionsphase.
Nach Applikation von Substanzen, die Effekte am biologisch aktiven Teil des Haares hervorrufen, könnte die Bestimmung der Veränderungen der Expression dieser Gene Aussagen über Haarwuchsstimulantien oder -inhibitoren liefern.
Die differentielle Genexpression wurde mittels quantitatitiver RT-PCR (Reverse-Transkription Polymerase Kettenreaktion) bestimmt. Nach Anzucht der dermalen Papillenzellen wurden diese für 24 h mit Dihydroquercetin in der Konzentration von 8,8 und 88 µM (entspricht Extrakt der Sibirischen Lerche von 0,003% und 0,0003%) inkubiert. Zur Durchführung der PCR wird zunächst mit Hilfe des RNeasy Mini Kits der Fa. Qiagen die RNA aus den dermalen Papillenzellen isoliert und mittels reverser Transkription in cDNA umgeschrieben. Bei der anschließenden PCR Reaktion, die mit Hilfe genspezifischer Primer für die jeweiligen haarrelevanten Gene durchgeführt wird und die der Amplifikation der gesuchten Genabschnitte dient, wird die Bildung der PCR-Produkte online über ein Fluoreszenzsignal detektiert. Das Fluoreszenzsignal ist dabei proportional zur Menge des gebildeten PCR-Produktes. Je stärker die Expression eines bestimmten Gens ist, desto größer ist die Menge an gebildetem PCR-Produkt und um so höher ist das Fluoreszenzsignal.
Zur Quantifizierung der Genexpression wird die unbehandelte Kontrolle gleich 1 gesetzt und die Expression der zu bestimmenden Gene darauf bezogen (x-fache Expression). Dabei werden Werte, die größer/gleich der 2fachen Expression oder kleiner/gleich der 0.5fachen Expression der unbehandelten Kontrolle sind als signifikant differentiell exprimiert eingestuft. Werte, die größer/gleich der 1,6fachen Expression oder kleiner/gleich der 0.66fachen Expression der unbehandelten Kontrolle sind als tendenziell differentiell exprimiert eingestuft.

**Tabelle 2: Einfluss von Dihydroquercetin auf die Expression Haarzyklus-regulierender Gene**

| | Konzentration | IGFBP3 | HGF | KGF |
|---|---|---|---|---|
| | [µM] | Mittelwert | Mittelwert | Mittelwert |
| Unbehandelte Probe | | 1, 00 (0,13) | 1,00 (0,42) | 1,00 (0,22) |
| Dihydroquercetin | 88 | 0,63(0,13) | 3,62(2,11) | 2,18 (0,27) |
| Dihydroquercetin | 8,8 | 0,72 (0,04) | 2,90 (1,18) | 2,71 (0,36) |

Die 24stündige Behandlung mit Dihydroquercetin führt in den untersuchten dermalen Papillenzellen zur signifikanten Induktion der Genexpression von KGF und tendenziell von HGF. Ausgehend von dem biologischen Potential von HGF und KGF kann anhand dieses Expressionsprofils auf positive Effekte hinsichtlich des Haarwachstums und einer Verbesserung der Haarstruktur rückgeschlossen werden. Zusätzlich führt Dihydroquercetin zur tendenziellen verminderten Expression von IGFBP-3. Im biologischen System führt dies zu einer verminderten Synthese des Insuline-like Growth Factor Binding Proteins, wodurch der Insulin-ähnliche Wachstumsfaktor an der Haarwurzel besser verfügbar ist. Die hier erhobenen Gen-Profile deuten auf Haarwuchs stimulierende und den vorzeitigen Haarausfall vermindernde Effekte hin.

### Beispiel 9: Nachweis der Freisetzung des Keratinozyten Wachstumsfaktor unter Verwendung dermaler Papillenzellen

Der Keratinozyten Wachstumsfaktor Factor (KGF) ist ein äußerst wichtiger Regulator des Haars. Bei einer potentiell wachstumsfördernden und das Haar stärkenden Aktivsubstanz kann von einer Steigerung der ins Medium ausgeschütteten Faktoren ausgegangen werden. Die Ausschüttung von KGF kann mit Hilfe von kommerziell erhältlichen ELISA-Kits quantifiziert werden. Dazu wurden die dermalen Papillenzellen über 24 Stunden mit Dihydroquercetin in der Konzentration von 8,8 und 88 µM (entspricht Extrakt der Sibirischen Lerche von 0,003% und 0,0003%) inkubiert und die Konzentration des Wachstumsfaktors im Medium in beschriebener Weise quantifiziert.

**Tabelle 3: Relative Ausschüttung von KGF in [%] nach Behandlung der dermalen Papillenzellen im Vergleich zur unbehandelten Probe.**

| | Konzentration | KGF |
|---|---|---|
| | [µM] | Mittelwert [%] |
| Unbehandelte Probe | | 100 (10,4) |
| Dihydroquercetin | 88 | 176 (16,7) |
| Dihydroquercetin | 8,8 | 173 (17,6) |

Die Behandlung mit Dihydroquercetin in der Konzentration von 8,8 und 88 µM (entspricht Extrakt der Sibirischen Lerche von0,003 und 0,0003%) führte bei dermalen Papillenzellen zu einer signifikant erhöhten Freisetzung von KGF. Die hier nachgewiesene erhöhte Freisetzung beruht auf der gesteigerten Expression des Gens für KGF.

KGF wird von der dermalen Papille, dem Steuerzentrum des Haarfollikels, ausgeschüttet, und reguliert die Proliferation von Haarschaftkeratinozyten. Eine erhöhte Proliferation der Haarschaftkeratinozyten kann wiederum zu einer positiven Beeinflussung der Haardicke führen.

### Beispiel 10: Nachweis der Freisetzung von Wachstumsfaktor unter Verwendung von organotypischen Haarfollikelzellkulturen

Hepatocyte Growth Factor (HGF) und Keratinocyte Growth Factor (KGF) sind wichtige Wachstumsfaktoren, die von der dermalen Papille ausgeschüttet werden, um die Proliferation der Haarschaftkeratinozyten, die für die Synthese der Haarkeratine verantwortlich sind, zu steuern. Sie sind außerdem charakteristische Marker für die Anagenphase, in der auch die Keratinsynthese maximal ist. Darüber hinaus ist zu beachten, dass bei der Haaralterung das Proliferationsvermögen der Haarfollikelzellen abnimmt. Die Ausschüttung von HGF sowie KGF kann mit Hilfe von kommerziell erhältlichen ELISA-Kits quantifiziert werden.
Dazu werden organotypische Haarfollikelzellkulturen über 72h mit Dihydroquercetin in der Konzentration 88 µM (entspricht Extrakt der Sibirischen Lerche von 0,003%) inkubiert und die Konzentration der Wachstumsfaktoren im Medium in beschriebener Weise bestimmt.

**Tabelle 4: Relative Ausschüttung von HGF und KGF in [%] nach Behandlung von organotypischen Haarfollikelzellkulturen im Vergleich zu unbehandelten Kontrollen.**

| | | KGF | HGF |
|---|---|---|---|
| | Konzentration | Mittelwert [%] | Mittelwert [%] |
| Unbehandelte Probe | | 100 (14) | 100 (11) |
| Dihydroquercetin | 88 µM | 126 (7) | 142 (15) |

Die Behandlung mit Dihydroquercetin in der Konzentration 88 µM (entspricht Extrakt der Sibirischen Lerche von 0,003%) führte unter Verwendung des organotypischen Haarmodells zu einer Aktivierung der Freisetzung von KGF. Die hier nachgewiesene erhöhte Freisetzung beruht auf der gesteigerten Synthese der dermalen Papillenzellen wie in Beispiel 9 gezeigt. Wie erwähnt kann die erhöhte Ausschüttung von KGF und die derart aktivierte Proliferation der Haarschaftkeratinozyten zu einer positiven Beeinflussung der Haardicke führen. Überraschenderweise wurde hierbei auch eine erhöhte Freisetzung des Hepatozyten Wachstumsfaktor (HGF) nachgewiesen werden. Dies beruht auf der interzellulären Kommunikation zwischen den dermalen Papillenzellen und den dermalen Fibroblasten, die ebenfalls im organotypischen Modell enthalten sind. HGF ist maßgeblich an der Regulation des Haarzyklus beteiligt. Eine hohe Konzentration im Haarfollikel wird in der Phase des aktiven Haarwuchses (Anagen) detektiert. Weitere Untersuchungen zeigten, dass neben der Aktivierung des Haarfollikel-Wachstums HGF zu einer signifikanten Verzögerung der Regressionsphase der Haarfollikel (Katagen) führt. Dies wiederum führt zu einer signifikanten Verlängerung der Haarwachstumsphase und einer Verminderung des Haarausfalls. Darüber hinaus steuert HGF die Zellproliferation in der Haarmatrix. Durch die gesteigerte Proliferation kann die Haarmatrix verdickt und so potentiell das Volumen der Haarfaser beeinflusst werden.

### Beispiel 11: Steigerung der Schichtdicke

Physiologische Effekte der Behandlung mit potentiellen Wirkstoffen hinsichtlich einer Verdickung des Haares kann anhand einer Steigerung der Schichtdicke der "Outer Root Sheath" Keratinozyten im organotypischen Zellkulturmodell bestimmt werden. Dazu werden jeweils drei Modelle nach 72 stündiger Inkubation mit Wirkstoff oder mit Referrenzmedium histologisch analysiert: Fixation in 4 % Paraformaldehyd, Färbung mittels Propidiumiodid und mikroskopische Analyse. Im Folgenden wird die Dicke der ORS Keratinozytenschicht des organptypischen Modells randomisiert vermessen und die Ergebnisse werden in Relation zur unbehandelten Kontrolle dargestellt.

**Tabelle 5: Steigerung der Schichtdicke nach Behandlung der organptypischen Zellkultur mit Dihydroquercetin in der Konzentration von 88 µM (entspricht Extrakt der Sibirischen Lerche von 0,003%) in % im Vergleich zur unbehandelte Probe**

| | Mittelwert |
|---|---|
| Kontrolle | 100 (7) |
| Dihydroquercetin | 111 (4) |

Überraschenderweise konnte nach Behandlung mit Dihydroquercetin eine tendenzielle Steigerung der ORS Keratinozytenschichtdicke um 11 % festgestellt werden. Die hier nachgewiesene Steigerung der Schichtdicke ist die Konsequenz der in Beispiel 9 und 10 beschriebenen Erhöhung der KGF Ausschüttung nach Behandlung mit Dihydroquercetin. Die Erhöhung der Schichtdicke beruht auf einer gesteigerten Proliferation der Haarschaftkeratinozyten und führt in Konsequenz zu einer Verdickung des Haarschaftes. Dies wiederum trägt somit zur Erhöhung des Haarvolumens bei.

### Beispiel 12: Nachweis der Aktivierung der Keratinsynthese

Die Festigkeit der Haare und die Haarstruktur ist im Wesentlichen von der Zusammensetzung spezieller haarspezifischer Strukturproteine abhängig, den Haarkeratinen. Durch die Veränderung der Zusammensetzung dieser spezifischen Proteine wird auf biologischer Ebene Einfluss auf die Haarstruktur genommen.
Die Expression verschiedener Haarkeratine organotypischen Modellen kann mit Hilfe des in Beispiel 8 beschriebenen quantitativen Real-Time-PCR-Verfahrens untersucht werden. Zur Quantifizierung der Genexpression wird die Lösungsmittelkontrolle gleich 1 gesetzt und die Expression der zu bestimmenden Gene darauf bezogen (x-fache Expression). Dabei werden Werte, die größer/gleich der 2fachen Expression oder kleiner/gleich der 0,5fachen Expression der unbehandelten Kontrolle sind als signifikant differentiell exprimiert eingestuft.

**Tabelle 6: Haarkeratinexpression nach Behandlung organotypischer Zellkulturen im Vergleich zur unbehandelten Probe, 24h nach Applikation**

| | hHa3-I | hHa4 |
|---|---|---|
| Unbehandelte Probe | 1,0(0,05) | 1,0 (0,02) |
| Dihydroquercetin [88 µM] | 4,1 (1,5) | 4,1 (1,6) |

Demnach führt Dihydroquercetin in der Konzentration von 88 µM (entspricht Extrakt der Sibirischen Lerche von 0,003%) in organotypischen Zellkulturen 24h nach Applikation zu einer Steigerung der Keratinexpression.

## Patentansprüche

1. Haarbehandlungsmittel, enthaltend mindestens eine Verbindung ausgewählt aus der Gruppe der Quercetine.

2. Mittel nach Anspruch 1, **dadurch gekennzeichnet, dass** die Quercetine ausgewählt sind aus der Gruppe, umfassend 3,3',4',5,7-Pentahydroxyflavanone, 3,3',4',5,7-Pentahydroxyflavon 3-β-glucoside (Isoquercitrin), Quercetin-7-methylether, Quercetin-3',7-dimethylether, Quercetin-3-*O*-β-D-glucofuranoside, Quercetin-7-*O*-glucoside, Quercetin-4'-*O*-glucoside, Quercetin-3-*O-*galactoside, Quercetin-3-D-xyloside, Quercetin- 3-rhamnoside (Quercitrine), Quercetin-3-rutinoside, Dihydroquercetine und Mischungen daraus.

3. Mittel nach Anspruch 2, **dadurch gekennzeichnet, dass** die Quercetine Quercetin- haltige Extrakte sind.

4. Mittel nach einem der Ansprüche 1 bis 3, **dadurch gekennzeichnet, dass** die Quercetine Dihydroquercetin-haltige Extrakte sind.

5. Mittel nach einem der Ansprüche 1 bis 4, **dadurch gekennzeichnet, dass** das Mittel 0,00001 bis 5 Gew.%, vorzugsweise 0,0001 bis 2,5 Gew.%, besonders bevorzugt 0,0001 bis 1 Gew.-% und insbesondere 0,00025 bis 0,5 Gew.-% bezogen auf das gesamte Mittel Quercetine oder Quercetin-haltige Extrakte enthält.

6. Mittel nach einem der Ansprüche 1 bis 5, **dadurch gekennzeichnet, dass** das Mittel mindestens einen weiteren Stoff aus der Gruppe der Vitamine oder Vitaminvorstufen enthält.

7. Mittel nach einem der Ansprüche 1 bis 6, **dadurch gekennzeichnet, dass** es sich bei den Vitaminen und Vitaminvorstufen um Panthenol oder Pantolacton handelt.

8. Mittel nach einem der Ansprüche 1 bis 7, **dadurch gekennzeichnet, dass** es weiterhin mindestens einen Emulgator und/oder mindestens einen Dialkylether und/oder ein Gemisch dieser Stoffe enthält.

9. Mittel nach einem der Ansprüche 1 bis 8, **dadurch gekennzeichnet, dass** der Emulgator ausgewählt ist aus Polyethylenglycolalkylethern und/oder ethoxylierten Glycerinestern.

10. Mittel nach einem der Ansprüche 1 bis 9, **dadurch gekennzeichnet, dass** das Mittel zusätzliche Bestandteile enthält, ausgewählt aus der Gruppe, umfassend Proteinhydrolysate, filmbildende Substanzen, weitere haarwuchsstimulierende Wirkstoffe, nichtionogene Tenside, anionische Tenside, zwitterionische Tenside, ampholytische Tenside, nichtionische Polymere, Verdickungsmittel, Parfümöle, Farbstoffe, Lichtschutzmittel, Antioxidantien, Antischuppenwirkstoffe, Treibmittel, Reduktionsmittel.

11. Mittel nach einem der Ansprüche 1 bis 10, **dadurch gekennzeichnet, dass** es weiterhin kationische Tenside ausgewählt aus quartären Ammoniumverbindungen, Esterquats und Amidoaminen enthält.

12. Mittel nach einem der Ansprüche 1 bis 11, **dadurch gekennzeichnet, dass** es sich um ein Shampoo, ein Haarnachspülmittel, ein Haarwasser, eine Haarkur, eine Haarcreme, eine Haarlotion, ein Haarspray oder eine Haartinktur handelt.

13. Verwendung eines Mittels nach einem der Ansprüche 1 bis 12 zur Vitalisierung von Haaren, Reaktivierung der Haarwurzel, Aktivierung von Haarfollikeln, Haarverdickung, Förderung oder Verstärkung des Haarwachstums, zur Haarkonditionierung, zur Verminderung von Haarausfall und/oder zur Behandlung von Haarausfall.

14. Verfahren zur Vitalisierung von Haaren, Reaktivierung der Haarwurzel, Aktivierung von Haarfollikeln, Förderung oder Verstärkung des Haarwachstums, zur Haarkonditionierung, zur Verminderung von Haarausfall und/oder zur Behandlung von Haarausfall, **dadurch gekennzeichnet, dass** man ein Mittel nach einem der Ansprüche 1 bis 12 auf die Haare, Kopfhaut, behaarte Haut bzw. auf Hautstellen, wo der Haarwuchs seit längerem eingestellt ist, wo jedoch ein Haarwuchs gewünscht wird, aufbringt.
